# EUROPEAN PATENT SPECIFICATION

(11) **EP 1 609 792 B1**
(45) Date of publication and mention of the grant of the patent: **10.12.2008**
(21) Application number: 05012621.8
(22) Date of filing: 13.06.2005
(51) Int. Cl.: C07D 471/04

(54) **A process for the preparation of imiquimod and intermediates thereof**
Verfahren zur Herstellung von Imiquimod und dessen Zwischenverbindungen
Procedé de préparation d'Imiquimod et ses intermédiaires

(30) Priority: 24.06.2004 IT MI20041282
(43) Date of publication of application: 28.12.2005
(73) Proprietor: Dipharma Francis S.r.l., 20021 Baranzate di Bollate MI (IT)
(72) Inventor: Razzetti, Gabriele, 20099 Sesto S. Giovanni (MI) (IT); Porta, Eleonora, 22036 Erba (CO) (IT)
(74) Representative: Bianchetti, Giuseppe

(56) References cited:
- WO-A-92/15581
- WO-A1-97/48704
- US-A- 4 689 338

## Description

### FIELD OF THE INVENTION

The present invention relates to a novel process for the preparation of Imiquimod and novel hydroxylamine and hydrazine derivatives useful as intermediates for its preparation.

Imiquimod, 4-amino-1-isobutyl-1H-imidazo[4,5-c]quinoline (A), is an antiviral and immunomodulating medicament, disclosed in US 4,689,338.

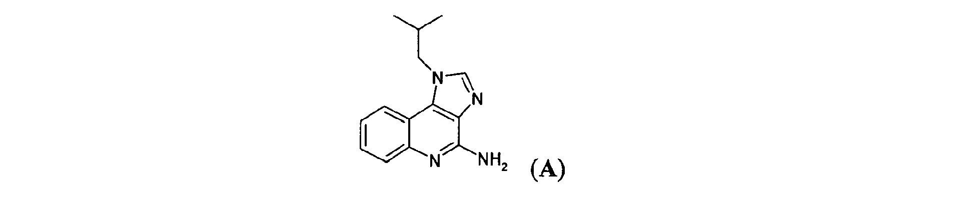

### TECHNOLOGICAL BACKGROUND

A number of synthetic methods for the preparation of Imiquimod are known, some of which make use of the corresponding 4-chloro-1H-imidazo[4,5-c]quinoline **(I)** as an intermediate. One of these methods is disclosed in US 4,689,338 which comprises as the last step the following reaction:

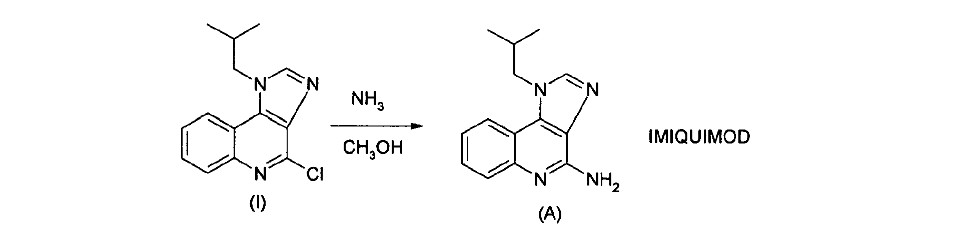

The chlorine atom at the 4- position in the intermediate (I) is replaced by an amino group by treatment with ammonia for 18 hours at 155°C in a steel autoclave. Said reaction is carried out under conditions involving temperatures and pressures which require the use of specific industrial equipment. WO 97/48704 discloses a process for the preparation of imidazoquinolinamines comprising the reaction between 6H-imidazo[4,5-c]tetrazolo[1,5-a]quinoline with triphenylphosphine and hydrolysis the product thereof; the removal of triphenylphosphine from the reaction mixture is, however, troublesome.
WO 92/15581 discloses a process for preparing 1-substituted-1H-inidazo[4,5-c]quinolin-4-amines which comprises the reaction of a 1-substituted -1H-imidazo[4,5-c]quinoline-5N-oxide with an isocyanate ad hydrolysisg the product thereof.There is therefore the need for alternative methods for the preparation of Imiquimod, which are more industrially suitable.

### SUMMARY OF THE INVENTION

It has now surprisingly been found that the intermediate (I), described above, can be conveniently transformed into Imiquimod by reaction with hydrazine, hydroxylamine or a derivative thereof. The novel process for the preparation of Imiquimod does not involve the use of potentially dangerous or problematic reaction conditions. The advantages of this process will be appreciated by the following description.

### DETAILED DISCLOSURE OF THE INVENTION

An object of the invention is a process for the preparation of Imiquimod, 4-amino-1-isobutyl-1H-imidazo[4,5-c]quinoline, comprising the reaction of 4-chloro-1-isobutyl-1H-imidazo[4,5-c]quinoline, of formula **(I)** 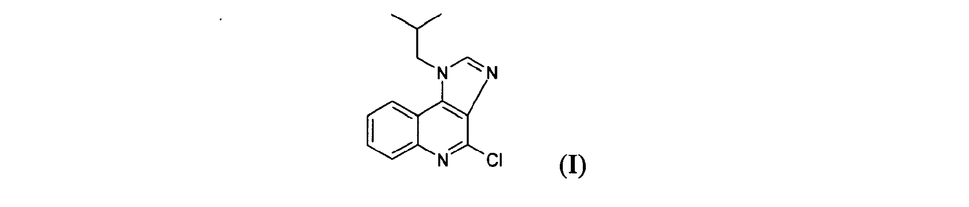 with a compound of formula **(II)**

NH₂-X **(II)**

wherein X is an -OR or -NR₁R₂ group in which R is hydrogen, a C₁-C₆ alkyl, aryl-C₁-C₄ alkyl, aryl or -SO₃H (sulfonic) group; and each of R₁ and R₂ is independently hydrogen, a C₁-C₆ alkyl, aryl-C₁-C₄ alkyl, aryl or -SO₂R₃ group, wherein R₃ is an aryl group; and, if necessary, the reaction with a reducing agent.

A R, R₁ or R₂ C₁-C₆ alkyl group, which may be straight or branched, is preferably a C₁-C₄ alkyl group, such as methyl, ethyl, propyl or isopropyl, in particular methyl or ethyl.

An R, R₁ or R₂ aryl-C₁-C₄ alkyl group, wherein the alkyl moiety can be straight or branched, is for example phenyl-C₁-C₄ alkyl, preferably benzyl or phenylethyl, wherein the phenyl ring can be optionally substituted with one to three substituents independently selected from hydroxy, C₁-C₄ alkoxy, for example methoxy, ethoxy or propoxy; thio, C₁-C₄ alkyl-thio, for example methylthio or ethylthio; nitro, cyano, halogen, for example chlorine, bromine or iodine.

An R, R₁, R₂ or R₃ aryl group is for example a phenyl or naphthyl group, in particular optionally substituted phenyl with one to three substituents independently selected from hydroxy, alkoxy-C₁-C₄, for example methoxy, ethoxy or propoxy; thio, C₁-C₄ alkyl-thio, for example methylthio or ethylthio; nitro, cyano and halogen, for example chlorine, bromine or iodine.

In a compound of formula **(II),** when X is -OR, R is preferably hydrogen, a C₁-C₄ alkyl or -SO₃H group, and when X is -NR₁R₂ then R₁ and R₂ are preferably hydrogen.

"Compound of formula **(II)"** means both the compound of formula **(II)** as such and a salt or hydrated form thereof.

A salt of a compound of formula **(II)** is for example an addition salt with a mineral or organic acid, such as hydrochloric, hydrobromic, sulfuric, phosphoric, acetic, oxalic, fumaric, methanesulfonic or ethanesulfonic acid, preferably hydrochloric or sulphuric acid, in particular hydrochloric acid. A compound (II) can be used also in a hydrated form, for example hydrazine hydrate.

The stoichiometric ratio of the compound (II) to a compound (I), as defined above, ranges from 0.5 to 10, preferably from 1 to 5.

The reaction between a compound (I) and a compound (II), as defined above, can be optionally carried out in the presence of solvent. Said solvent is preferably an organic solvent or mixtures thereof with water. Typically, an aromatic hydrocarbon, such as toluene or xylene; a chlorinated solvent, such as dichloromethane, dichloroethane, tetrachloroethylene, chlorobenzene or dichlorobenzene; an ester solvent, such as ethyl acetate, isopropyl acetate or butyl acetate; an ether solvent, such as tetrahydrofuran, dioxane, ethyl ether or butyl ether; an alcoholic solvent such as methanol, ethanol, isopropanol; a dipolar aprotic solvent such as acetonitrile, dimethylformamide, dimethylacetamide, dimethylsulfoxide or N-methylpyrrolidone; or a mixture of said organic solvents and/or water. Particularly preferred are methanol, ethanol or a mixture of ethanol and water.

The reaction can be carried out at a temperature ranging from about 0°C to the reflux temperature of the reaction mixture, preferably from about 30°C to the reflux temperature.

Furthermore, the reaction can be optionally carried out in the presence of a basic agent, for example an organic or inorganic base. Said basic agent serves to neutralize the acidity formed during the substitution reaction with chlorine of the compound (I) and moreover, if necessary, to deprotect the compound of formula (II) if present in the salified form. Examples of organic or inorganic bases are trisubstituted amines, in particular triethylamine or diisopropylethylamine, sodium acetate or potassium carbonate.

If necessary, the reaction with a reducing agent can be carried out using an agent selected from, for example, tin(II) chloride, zinc in hydrochloric acid, sodium thiosulfate, potassium iodide, thiourea and Pd on carbon together with a hydrogen donor, such as sodium formate, ammonium formate, potassium formate, formic acid, cyclohexene or limonene; or a derivative of formula **(IIa) NH₂-X',** or a salt or hydrated form thereof, wherein X' is NH₂ or OR', in which R' is hydrogen, C₁-C₄ alkyl or SO₃H, preferably hydrogen.

A salt of a compound of formula **(IIa)** or a hydrated form thereof are the same as reported above in connection with a compound of formula **(II).**

The reaction between a compound (I) and a compound (II), in fact, can optionally be carried out stepwise, namely comprising the formation of a compound **(III)** or **(IIIa),** 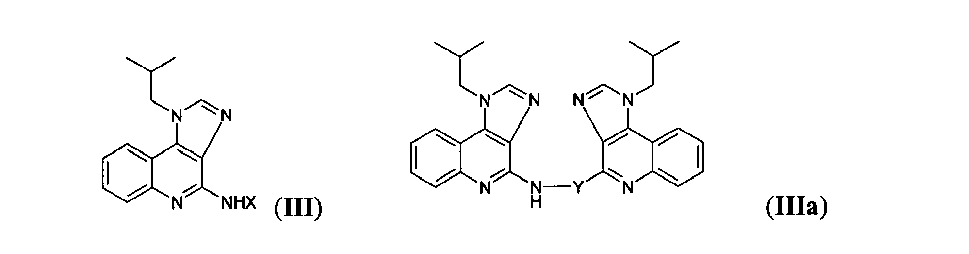 wherein X is an -OR, -NHR₁ or -NR₁R₂ group, in which R is hydrogen, a C₁-C₆ alkyl, aryl-C₁-C₄ alkyl, aryl or SO₃H (sulfonic) group and R₁ and R₂ are independently a C₁-C₆ alkyl, aryl-C₁-C₄ alkyl, aryl or SO₂R₃, in which R₃ is an aryl group;; and Y is -O- or -NH-; and following reduction.

The reaction between a compound **(I)** and a compound **(II)** to afford a compound **(III)** or **(IIIa)** can be carried out analogously to what reported above.

The reduction of a compound **(III)** or **(IIIa),** which can optionally be isolated, to give Imiquimod, can be carried out using a reducing agent as defined above, such as zinc in hydrochloric acid, sodium thiosulfate or Pd/C in the presence of sodium formate as a hydrogen donor.

When in a compound **(II)** X is different from -OH, then its reaction with a compound (I) is preferably carried out stepswise and the resulting compound (III), in which X is different from -OH, is reduced by means of a reducing agent as defined above, preferably selected from tin(II) chloride, zinc in hydrochloric acid, sodium thiosulfate, potassium iodide, thiourea or Pd/C together with sodium formate as hydrogen donor.

More preferably, when in a compound of formula (III) X is -OR, and R is different from hydrogen, the reducing agent is selected from zinc in hydrochloric acid, sodium thiosulfate, potassium iodide, thiourea; whereas when X is -NH₂, the reducing agent is Pd/C together with sodium formate.

The reduction reaction can be carried out adding the reducing agent to the reaction mixture containing the derivative **(III)** or **(IIIa),** or preferably directly reacting the compound **(I)** with a compound **(II)** in the presence of the reducing agent.

The compounds **(III)** and **(IIIa),** as defined above, are novel and are a further object of the invention.

Preferred examples of compounds **(III)** and **(IIIa)** are:
- N-(1-isobutyl-1H-imidazo[4,5-c]quinolin-4-yl)-O-methylhydroxylamine;
- N-(1-isobutyl-1H-imidazo[4,5-c]quinolin-4-yl)-O-sulfonyl-hydroxylamine;
- N,N'-Bis-(1-isobutyl-1H-imidazo[4,5-c]quinolin-4-yl)-hydrazine.

The following examples illustrate the invention.

### Example 1: Synthesis of N-(1-Isobutyl-1H-imidazo[4,5-c]quinolin-4-yl)-O-methyl-hydroxylamine (III).

A round-bottom three-necked flask equipped with condenser, magnetic stirrer, thermometer, is loaded with 4-chloro-1-isobutyl-1H-imidazo[4,5-c]quinoline (5.0 g, 0.019 mol), O-methyl hydroxylamine hydrochloride (3.2 g, 0.038 mol), sodium acetate (3.1 g, 0.038 mol), and an ethanol/water 2:1 v/v mixture (60 ml). The resulting mixture is then refluxed. After 14 hours, a 30% NaOH aqueous solution is added to pH 9 and the mixture is extracted with methylene chloride (3x50 ml). The combined organic phases are dried over sodium sulfate and evaporated to a residue. The resulting white solid is dried under vacuum at 50°C. Weight = 4.35 g; Yield = 75%.
¹HNMR (300 M Hz, DMSO-d6): δ (ppm) 8.16 (s, 1H), 7.82 (d, 1H, J = 8.2 Hz), 7.80 (d, 1H, J = 8.2 Hz), 7.40 (t, 1H, J = 8.2 Hz), 7.22 (t, 1H, J = 8.2 Hz), 4.35 (d, 2H, J = 7 Hz), 3.85 (s, 3H), 2.05 (m, 1H), 0.88 (d, 6H, J = 7 Hz).

### Example 2: Synthesis of N-(1-Isobutyl-1H-imidazo[4,5-c]quinolin-4-yl)-O-sulfonyl-hydroxylamine (III).

A round-bottom three-necked flask equipped with condenser, magnetic stirrer, thermometer, is loaded with O-hydroxylamino sulfonic acid (4.8 g, 0.042 mol), methanol (30 ml), sodium acetate (3.1 g, 0.038 mol) and finally 4-chloro-1-isobutyl-1H-imidazo[4,5-c]quinoline (10.0 g, 0.038 mol). The mixture is heated to 40°C and left under stirring at this temperature for 24 hours. After completion of the reaction, the precipitate is filtered with suction and washed with water, then dried under vacuum at 50°C to obtain a white solid weighing 9.5 g (yield = 74%).
¹HNMR (300 M Hz, DMSO-d6): δ (ppm) 8.7 (s, 1H), 8.16 (d, 1H, J = 8.2 Hz), 8.07 (d, 1H, J = 8.2 Hz), 7.62 (t, 1H, J = 8.2 Hz), 7.48 (t, 1 H, J = 8.2 Hz), 4.9 (bs, 1H), 4.45 (d, 2H, J = 8 Hz), 3.15 (s, 1H), 2.13 (m, 1H), 0.89 (d, 6H, J = 8 Hz).

### Example 3: Synthesis of Imiquimod starting from N-(1-isobutyl-1H-imidazo[4,5-c]quinolin-4-yl)-O-methyl-hydroxylamine

A round-bottom three-necked flask equipped with condenser, magnetic stirrer, thermometer, is loaded with N-(1-isobutyl-1H-imidazo[4,5-c]quinolin-4-yl)-O-methyl-hydroxylamine (0.2 g, 0.74 mmol) and ethanol (15 ml). 37% HCl (1 ml) and zinc powder (48 mg, 0.74 mmol) are added under stirring. After stirring for one hour at room temperature the reaction is completed. The mixture is concentrated to half the volume, alkalinised with 50% w/w NaOH to pH 9-10 and left under stirring for 30 minutes. The resulting solid is filtered and dried under vacuum in a static dryer at 50°C. (0.12 g, yield 70%).
¹HNMR (300 M Hz, DMSO-d6): δ (ppm): 8.16 (s,1H), 8.0 (d,1H, J = 8.2 Hz), 7.61 (d,1H, J = 8.2 Hz), 7.42 (t,1H, J = 8.2 Hz), 7.26 (t,1H, J = 8.2 Hz), 6.54 (bs, 2H), 4.39(d, 2H, J = 7.5 Hz), 2.18 (m,1H), 0.91(d, 6H, J = 7.5 Hz).

### Example 4: Synthesis of Imiquimod

A round-bottom three-necked flask equipped with condenser, magnetic stirrer, thermometer, is loaded with 4-chloro-1-isobutyl-1H-imidazo[4,5-c]quinoline (5.0 g, 0.019 mol), hydroxylamine hydrochloride (2.6 g, 0.038 mol), sodium acetate (3.1 g, 0.038 mol), an ethanol/water 2:1 v/v mixture (60 ml). The reaction mixture is refluxed for 12 hours then left to cool at room temperature. The precipitated solid (3 g, 0.010 mol) is filtered with suction and dried under vacuum at 50°C to constant weight. The resulting solid is then dissolved in water (6 ml) and added with sodium acetate (1 g, 0.012 mol). The precipitated product is filtered and dried under vacuum in a static dryer at 50°C. Weight = 2.4 g, Yield 52%.

### Example 5: Synthesis of N-(1-Isobutyl-1H-imidazo[4,5-c]quinolin-4-yl)-hydrazine (III).

A round-bottom three-necked flask equipped with condenser, magnetic stirrer, thermometer, is loaded with 4-chloro-1-isobutyl-1H-imidazo[4,5-c]quinoline (5.0 g, 0.019 mol), hydrazine hydrate (3.8 g, 0.076 mol), ethanol (20 ml). The mixture is refluxed for 3 hours then left to cool at room temperature, diluted with 10 ml of a 15% ammonia aqueous solution. The precipitated solid is filtered with suction and dried under vacuum at 50°C, thereby obtaining 4.5 g of N-(1-isobutyl-1H-imidazo[4,5-c]quinolin-4-yl)-hydrazine in 92% molar yield.
¹HNMR (300 M Hz, DMSO-d6): δ (ppm): 8.19 (s,1H), 7.98 (d,1H, J = 8.1 Hz), 7.70 (d,1H, J = 8.1 Hz), 7.44 (t,1H, J = 8.1 Hz), 7.27 (t,1H, J = 8.1 Hz), 4.37 (d, 2H, J = 7.5 Hz), 2.15 (m,1H), 0.88 (d, 6H, J = 6.6 Hz).

Following the same procedure, using 0.009 mol of hydrazine hydrate, N,N'-Bis-(1-isobutyl-1H-imidazo[4,5-c]quinolin-4-yl)-hydrazine is obtained.

### Example 6: Synthesis of Imiquimod starting from N-(1-isobutyl-1H-imidazo[4,5-c]quinolin-4-yl)-hydrazine

A round-bottom three-necked flask equipped with condenser, magnetic stirrer, thermometer, is loaded with N-(1-isobutyl-1H-imidazo[4,5-c]quinolin-4-yl)-hydrazine (4.5 g, 0.018 mol), water (40 ml) 37% hydrochloric acid (1.8 g, 0.018 mol), 10% Pd-C (humid, 50% of water) (3.8 g, 0.0018 mol) and sodium formate (4.9 g 0.072 mol). The mixture is refluxed for 84 hours then left to cool at room temperature, diluted with 10 ml of a 5% HCl solution to pH<2; filtered through Celite and alkalinised with an ammonia aqueous solution to pH>8. The precipitated solid is filtered with suction and dried under vacuum in a static dryer at 50°C, thereby obtaining 4.3 g in 98% molar yield.

## Claims

1. A process for the preparation of 4-amino-1-isobutyl-1H-imidazo[4,5-c]quinoline, comprising the reaction of 4-chloro-1-isobutyl-1H-infidazo[4,5-c]quinoline, of formula **(I)** 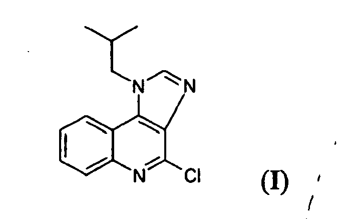 with a compound of formula **(II)**
NH₂-X **(II)**
wherein X is an -OR or -NR₁R₂ group in which R is hydrogen, a C₁-C₆ alkyl, aryl-C₁-C₄ alkyl, aryl or -SO₃H (sulfonic) group; and each of R₁ and R₂ is independently hydrogen, a C₁-C₆ alkyl, aryl-C₁-C₄ alkyl, aryl or -SO₂R₃ group, wherein R₃ is an aryl group; to give a compound **(III)** or **(IIIa),** 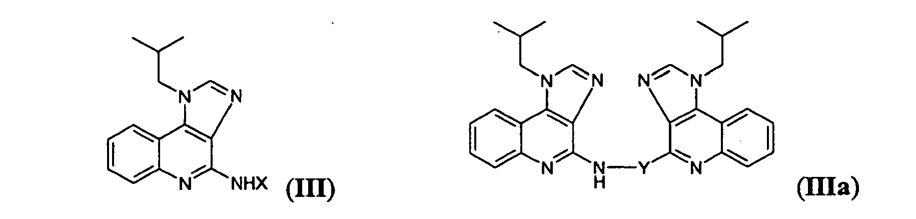 wherein Y is -0- or -NH- and X is as defined above;
followed by reaction with a reducing agent.

2. A process as claimed in claim 1, wherein in a compound **(II),** when X is -OR, R is hydrogen, a C₁-C₄ alkyl or -SO₃H group, and when X is -NR₁R₂, then R₁ and R₂ are hydrogen.

3. A process as claimed in claim 1 or 2, wherein the stoichiometric ratio of a compound **(II)** to the compound **(I)** ranges from 0.5 to 10.

4. A process as claimed in any one of claims 1 to 3, wherein the reaction is carried out in the presence of an organic solvent or mixtures thereof with water.

5. A process as claimed in claim 4, wherein the reaction is carried out in methanol, ethanol or in an ethanol/water mixture.

6. A process as claimed in any one of claims 1 to 5, wherein the reaction is carried out in the presence of a basic agent.

7. A process as claimed in claim 1, wherein the reducing agent is selected from tin(II) chloride, zinc in hydrochloric acid, sodium thiosulfate, potassium iodide, thiourea; or Pd/C together with a hydrogen donor; or a derivative **(IIa)** NH₂-X', or a salt or hydrated form thereof, wherein X' is NH₂ or OR', in which R' is hydrogen, C₁-C₄ alkyl or SO₃H.

8. A process as claimed in any one of claims 1-7, wherein when in a compound **(III)** X is different from OH, the reducing agent is selected from tin(II) chloride, zinc in hydrochloric acid, sodium thiosulfate, potassium iodide, thiourea and Pd/C together with sodium formate as a hydrogen donor.

9. A process as claimed in claim 8, wherein when in a compound **(III)** X is -OR, and R is different from hydrogen, the reducing agent is selected from zinc in hydrochloric acid, sodium thiosulfate, potassium iodide, thiourea; whereas, when X is -NH₂, the reducing agent is Pd/C together with sodium formate.

10. A process as claimed in claim 8 or 9, wherein the reduction is carried out reacting a compound **(I)** and a compound **(II)** in the presence of the reducing agent.

11. A compound having formula **(III)** or **(IIIa),** 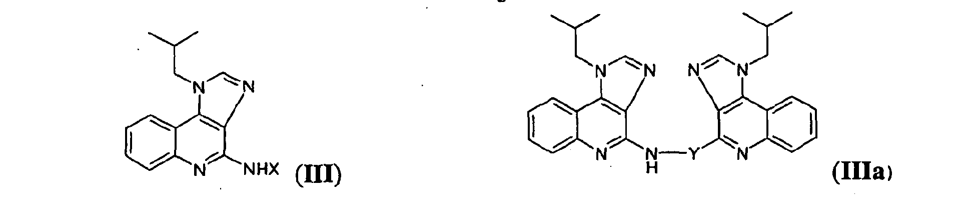 wherein X is an -OR, -NHR₁ or -NR₁R₂ group, in which R is hydrogen, a C₁-C₆ alkyl, aryl-C₁-C₄ alkyl, aryl or SO₃H (sulfonic) group and R₁ and R₂ are independently a C₁-C₆ alkyl, aryl-C₁-C₄ alkyl, aryl or SO₂R₃, in which R₃ is an aryl group; and Y is -O- or -NH-.

12. A compound of formula **(III)** or **(IIIa),** as claimed in claim 11, which is:
• N-(1-isobutyl-1H-imidazo[4,5-c]quinolin-4-yl)-O-methyl-hydroxylamine;
• N-(1-isobutyl-1H-imidazo[4,5-c]quinolin-4-yl)-O-sulfonyl-hydroxylamine;
or
• N,N'-Bis-(1-isobutyl-1H-imidazo[4,5-c]quinolin-4-yl)-hydrazine.

## Patentansprüche

1. Verfahren zur Herstellung von 4-Amino-1-isobutyl-1H-imidazo[4,5-c]chinolin, umfassend die Reaktion von 4-Chlor-1-isobutyl-1H-imidazo[4,5-c]chinolin der Formel (I) 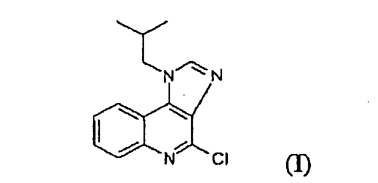 mit einer Verbindung der Formel (II)
NH₂-X **(II)**
worin X eine Gruppe -OR oder -NR₁R₂ ist, in der R Wasserstoff, eine C₁-C₆-Alkyl-, Aryl-C₁-C₄-alkyl-, Aryl- oder -SO₃H(Sulfon)-Gruppe ist; und jedes R₁ und R₂ unabhängig Wasserstoff, eine C₁-C₆-Alkyl-, Aryl-C₁-C₄-alkyl-, Aryl- oder -SO₂R₃-Gruppe ist, worin R₃ eine Arylgruppe ist;
um eine Verbindung der Formel (III) oder (IIIa) zu erhalten, 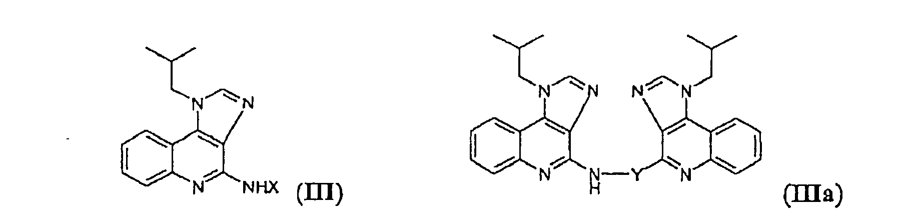 worin Y gleich -O- oder -NH- ist und X wie vorstehend angegeben definiert ist;
gefolgt von der Umsetzung mit einem Reduktionsmittel.

2. Verfahren nach Anspruch 1, wobei in einer Verbindung (II), wenn X gleich -OR ist, R gleich Wasserstoff, eine C₁-C₄-Alkyl- oder SO₃H-Gruppe ist, und wenn X gleich -NR₁R₂ ist, dann sind R₁ und R₂ gleich Wasserstoff sind.

3. Verfahren nach Anspruch 1 oder 2, wobei das stöchiometrische Verhältnis einer Verbindung (II) zu der Verbindung (I) im Bereich von 0,5 bis 10 liegt.

4. Verfahren nach einem der Ansprüche 1 bis 3, wobei die Reaktion in Gegenwart eines organischen Lösungsmittels oder Gemischen davon mit Wasser durchgeführt wird.

5. Verfahren nach Anspruch 4, wobei die Reaktion in Methanol, Ethanol oder in einem Ethanol/Wasser-Gemisch durchgeführt wird.

6. Verfahren nach einem der Ansprüche 1 bis 5, wobei die Reaktion in Gegenwart eines basischen Mittels durchgeführt wird.

7. Verfahren nach Anspruch 1, wobei das Reduktionsmittel ausgewählt ist aus Zinn(II)-chlorid, Zink in Salzsäure, Natriumthiosulfat, Kaliumiodid, Thioharnstoff; oder Pd/C in Kombination mit einem Wasserstoffdonor; oder einem Derivat (IIa) NH₂X', oder einem Salz oder einer hydratisierten Form davon, worin X' gleich NH₂ oder OR' ist, worin R' Wasserstoff, C₁-C₄-Alkyl oder SO₃H ist.

8. Verfahren nach einem der Ansprüche 1 bis 7, wobei, wenn in einer Verbindung (III) X von OH verschieden ist, das Reduktionsmittel ausgewählt ist aus Zinn(II)-chlorid, Zink in Salzsäure, Natriumthiosulfat, Kaliumiodid, Thioharnstoff und Pd/C in Kombination mit Natriumformiat als Wasserstoffdonor.

9. Verfahren nach Anspruch 8, wobei, wenn in einer Verbindung (III) X gleich -OR ist und R von Wasserstoff verschieden ist, das Reduktionsmittel ausgewählt ist aus Zink in Salzsäure, Natriumthiosulfat, Kaliumiodid, Thioharnstoff; wohingegen, wenn X gleich -NH₂ ist, das Reduktionsmittel Pd/C in Kombination mit Natriumformiat ist.

10. Verfahren nach Anspruch 8 oder 9, wobei die Reduktion durchgeführt wird durch Reaktion einer Verbindung (I) und einer Verbindung (II) in Gegenwart des Reduktionsmittels.

11. Verbindung der Formel (III) oder (IIIa) 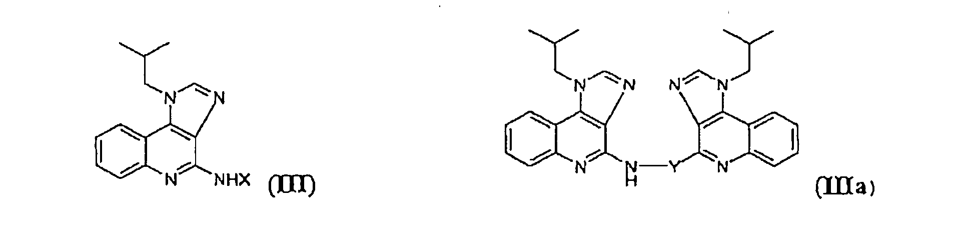 worin X eine Gruppe -OR,-NHR₁ oder -NR₁R₂ ist, in der R Wasserstoff, eine C₁-C₆-Alkyl-, Aryl-C₁-C₄-alkyl-, Aryl- oder -SO₃H(Sulfon)-Gruppe ist; und R₁ und R₂ unabhängig voneinander eine C₁-C₆-Alkyl-, Aryl-C₁-C₄-alkyl-, Aryl-, oder -SO₂R₃-Gruppe sind, in der R₃ ein Arylgruppe ist; und Y gleich -O- oder - NH- ist.

12. Verbindung der Formel (III) oder (IIIa) nach Anspruch 11, nämlich:
■ N-(1-Isobutyl-1H-imidazo[4,5-c]chinolin-4-yl)-O-methyl-hydroxylamin;
■ N-(1-Isobutyl-1H-imidazo[4,5-c]chinoliri-4-yl)-O-sulfonyl-hydroxylamin
oder
■ N,N'-Bis(1-isobutyl-1H-imidazo[4,5-c]chinolin-4-yl)-hydrazin;

## Revendications

1. Procédé pour la préparation de 4-amino-1-isobutyl-1H-imidazo[4,5-c]quinoléine, comprenant la réaction de 4-chlorc-1-isobutyl-1H-imidazo[4,5-c]quinoléine, de formule (1) 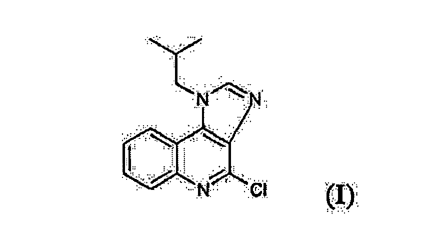 avec un composé de formule (II)
NH₂-X (II)
où X est un groupe -OR ou -NR₁R₂ dans lequel R est un atome d'hydrogène, un groupe alkyle en C₁-C₆, un groupe arylalkyle en C₁-C₄, un groupe aryle ou un groupe -SO₃H (sulfonique) ; et chacun de R₁ et R₂ est indépendamment un atome d'hydrogène, un groupe alkyle en C₁-C₆, un groupe arylalkyle en C₁-C₄, un groupe aryle ou un groupe -SO₂R₃, dans lequel R₃ est un groupe aryle ;
pour donner un composé (III) ou (IIIa), 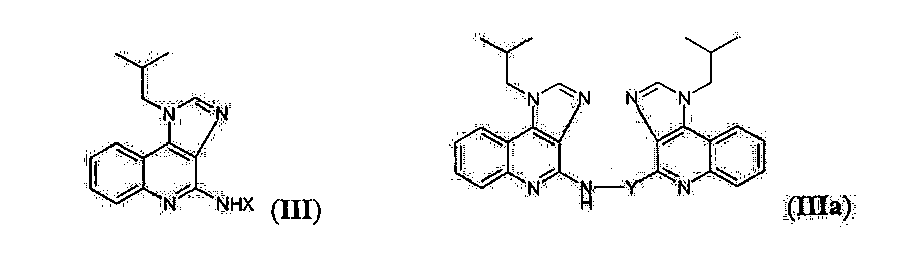 où Y est -O- ou -NH- et X est tel que défini ci-dessus ;
suivie par la réaction avec un agent de réduction.

2. Procédé selon la revendication 1, dans lequel dans un composé (II), lorsque X est -OR, R est un atome d'hydrogène, un groupe alkyle en C₁-C₄ ou un groupe -SO₃H, et lorsque X est -NR₁R₂, alors R₁ et R₂ sont un atome d'hydrogène.

3. Procédé selon la revendication 1 ou 2, dans lequel le rapport stoechiométrique d'un composé (II) au composé (I) est compris dans la plage allant de 0,5 à 10.

4. Procédé selon l'une quelconque des revendications 1 à 3, dans lequel la réaction est réalisée en présence d'un solvant organique ou de mélanges de celui-ci avec de l'eau.

5. Procédé selon la revendication 4, dans lequel la réaction est réalisée dans du méthanol, de l'éthanol ou dans mélange d'éthanol/eau.

6. Procédé selon l'une quelconque des revendications 1 à 5, dans lequel la réaction est réalisée en présence d'un agent basique.

7. Procédé selon la revendication 1, dans lequel l'agent de réduction est choisi parmi le chlorure d'étain(II), le zinc dans de l'acide chlorhydrique, le thiosulfate de sodium, l'iodure de potassium, la thiourée ; ou Pd/C avec un donneur d'hydrogène ; ou un dérivé (IIa) NH₂-X', ou un sel ou une forme hydratée de celui-ci, où X' est NH₂ ou OR', dans lequel R' est un atome d'hydrogène, un groupe alkyle en C₁-C₄ ou un groupe -SO₃H.

8. Procédé selon l'une quelconque des revendications 1 à 7, dans lequel lorsque dans un composé (III) X est différent de OH, l'agent de réduction est choisi parmi le chlorure d'étain (II), le zinc dans de l'acide chlorhydrique, le thiosulfate de sodium, l'iodure de potassium, la thiourée et Pd/C avec du formate de sodium en tant que donneur d'hydrogène.

9. Procédé selon la revendication 8, dans lequel lorsque dans un composé (III) X est -OR, et R est différent d'un atome d'hydrogène, l'agent de réduction est choisi parmi le zinc dans de l'acide chlorhydrique, le thiosulfate de sodium, l'iodure de potassium, la thiourée ; alors que lorsque X est -NH₂, l'agent de réduction est Pd/C avec du formate de sodium.

10. Procédé selon la revendication 8 ou 9, dans lequel la réduction est réalisée en faisant réagir un composé (I) et un composé (II) en présence de l'agent de réduction.

11. Composé ayant la formule (III) ou (IIIa), 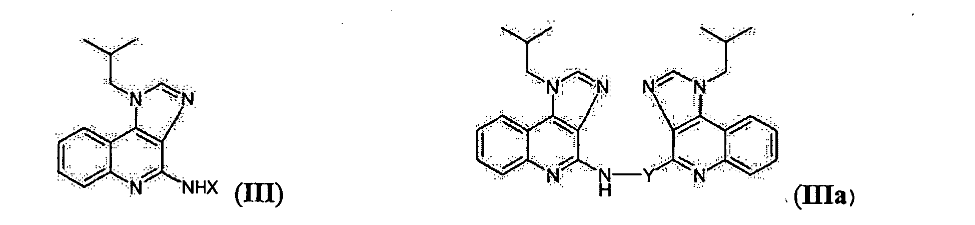 où X est un groupe -OR, -NHR₁ ou -NR₁R₂ dans lequel R est un atome d'hydrogène, un groupe alkyle en C₁-C₆, un groupe arylalkyle en C₁-C₄, un groupe aryle ou un groupe -SO₃H (sulfonique) et chacun de R₁ et R₂ est indépendamment un groupe alkyle en C₁-C₆, un groupe arylalkyle en C₁-C₄, un groupe aryle ou un groupe - SO₂R₃, dans lequel R₃ est un groupe aryle ; et Y est -O- ou -NH-.

12. Composé de formule (III) ou (IIIa), selon la revendication 11, qui est :
• la N-(1-isobutyl-1H-imidazo[4,5-c]quinoléin-4-yl)-O-méthyl-hydroxylamine ;
• la N-(1-isobutyl-1H-imidazo[4,5-c]quinoléin-4-yl)-O-sulfonyl-hydroxylamine ;
ou
• la N,N'-bis-(1-isobutyl-1H-imidazo[4,5-c]quinoléin-4-yl)-hydrazine.
